# EUROPEAN PATENT APPLICATION

(11) **EP 3 061 526 A1**
(43) Date of publication of application: **31.08.2016**
(21) Application number: 14856781.1
(22) Date of filing: 10.10.2014
(51) Int. Cl.: B01J 37/16, B01J 37/02, B01J 37/08, B01J 21/06

(54) **METHOD FOR PREPARING PHOTOCATALYST, AND PHOTOCATALYST PREPARED THEREBY**

(30) Priority: 22.10.2013 KR 20130126011
(71) Applicant: LG Hausys, Ltd., Seoul 150-721 (KR)
(72) Inventor: SEO, Joo-Hwan, Seoul 133-808 (KR); LEE, Dong-Il, Anyang-si Gyeonggi-do 431-761 (KR); LEE, Ju-Hyung, Uiwang-si Gyeonggi-do 437-837 (KR); JUNG, Seong-Moon, Daejeon 305-707 (KR)
(74) Representative: Prinz & Partner mbB
(86) International application number: PCT/KR2014/009542
(87) International publication number: WO 2015/060568

(57) **Abstract**

Disclosed is a method for preparing a photocatalyst, including: forming a porous film of the first metal oxide formed from a first metal oxide by a sol-gel process; heat-treating the porous film of the first metal oxide to crystallize the first metal oxide; dipping the porous film of the first metal oxide into a precursor solution of a second metal, followed by a photo-irradiation, such that ions of the second metal is to be penetrated inside pores of the porous film of the first metal oxide; and dipping the porous film of the first metal oxide containing the ions of the second metal inside the pores into a solution of alcohol, followed by a photo-irradiation, such that the ions of the second metal is to be reduced to form particles of the second metal inside the pores of the porous film of the first metal oxide.

## Description

### [Technical Field]

The present disclosure relates to a method for preparing a photocatalyst, and a photocatalyst prepared thereby.

### [Background Art]

Typical photocatalytic material, titanium oxide (TiO₂), is excellent in durability and wear resistance, and is safe and non-toxic, as well as low cost. However, because of its high band gap energy, titanium oxide absorbs only a light having a wavelength shorter than or equivalent to an ultraviolet radiation. Therefore, certain limitations exist in indoor applications for buildings, unlike exterior materials.

In this respect, lots of studies have been made in the development of a catalyst that is photo-active under a visible light and is capable of absorbing the visible light for indoor applications. However, no consistent trend was found in a number of cases studied, and particularly no proven performance results could be found in real living conditions.

### [Disclosure]

### [Technical Problem]

One aspect of the present disclosure is to provide a method for preparing a visible light-responsive photocatalyst whose performance is effective even under indoor light sources.

Another aspect of the present disclosure is to provide a photocatalyst prepared by the method described above.

### [Technical Solution]

In one embodiment of the present disclosure, provided is a method for preparing a photocatalyst, including: forming a porous film of the first metal oxide formed from a first metal oxide by a sol-gel process; heat-treating the porous film of the first metal oxide to crystallize the first metal oxide; dipping the porous film of the first metal oxide into a precursor solution of a second metal, followed by a photo-irradiation, such that ions of the second metal is to be penetrated inside pores of the porous film of the first metal oxide; and dipping the porous film of the first metal oxide containing the ions of the second metal inside the pores into a solution of alcohol, followed by a photo-irradiation, such that the ions of the second metal is to be reduced to form particles of the second metal inside the pores of the porous film of the first metal oxide.

The particles of the second metal formed inside the pores of the porous film of the first metal oxide may have an average particle diameter of about 1 nm to about 10 nm.

The first metal oxide contained in the porous film of the first metal oxide may include at least one selected from titanium oxide, tungsten oxide, zinc oxide, niobium oxide, and combinations thereof.

The second metal may include at least one metal selected from the group consisting of tungsten, chromium, vanadium, molybdenum, copper, iron, cobalt, manganese, nickel, platinum, gold, cerium, cadmium, zinc, magnesium, calcium, strontium, barium, radium, and combinations thereof.

The photocatalyst may have a weight ratio of the particles of the second metal to the porous film of the first metal oxide of about 0.1:99.9 to about 1:99.

The photocatalyst may be photoactive under a visible light having a wavelength range of about 380 nm to about 780 nm.

The porous film of the first metal oxide may be formed in a thickness of about 30 nm to about 100 nm.

The photo-irradiation may be carried out by UV irradiation.

In another embodiment of the present disclosure, provided is a photocatalyst prepared by the method for preparing a photocatalyst, including: the porous film of the first metal oxide; and the particles of the second metal formed inside the pores of the porous film of the first metal oxide.

The photocatalyst may be applicable to air cleaning, deodorizing, or antibacterial applications.

### [Advantageous Effects]

The photocatalyst is responsive to a visible light, and has superior photocatalytic efficiency.

### [Description of Drawings]

The above and other objects and features of the present disclosure will become apparent from the following descriptions, when taken in conjunction with the accompanying drawings, in which:
Fig. 1 shows a SEM image of a photocatalyst prepared in Example 1; and
Fig. 2 shows a SEM image of a photocatalyst prepared in Comparative Example 1.

### [Best Mode]

Hereinafter, embodiments of the present disclosure will be described in detail. However, it should be understood that the following embodiments are provided for illustrative purposes only and are not to be in any way construed as limiting the present disclosure. The scope and spirit of the present disclosure should be defined only by the accompanying claims and equivalents thereof.

In one embodiment of the present disclosure, there is provided a method for preparing a photocatalyst, which may include: forming a porous film of the first metal oxide formed from a first metal oxide by a sol-gel process; heat-treating the porous film of the first metal oxide to crystallize the first metal oxide; dipping the porous film of the first metal oxide into a precursor solution of a second metal, followed by a photo-irradiation, such that ions of the second metal is to be penetrated inside pores of the porous film of the first metal oxide; and dipping the porous film of the first metal oxide containing the ions of the second metal inside the pores into a solution of alcohol, followed by a photo-irradiation, such that the ions of the second metal is to be reduced to form particles of the second metal inside the pores of the porous film of the first metal oxide.

A method for preparing a photocatalyst prepared by the method for preparing the photocatalyst may include the porous film of the first metal oxide; and the particles of the second metal formed inside the pores of the porous film of the first metal oxide.

The first metal oxide for forming the porous film of the first metal oxide may be anything well known in the art, as long as it can be used asa photocatalyst. The metal for the particles of the second metal is not particularly limited as long as it is photoactive in a visible light region, and for example may include transition metals, noble metals, and the like.

The photocatalyst, formed by the method described above in such a manner that the particles of the second metal are doped into the first metal oxide, may be photoactive in the visible light region.

Thus, since the photocatalyst contains the particles of the second metal which is photoactive in a region of a visible light, it can be active even in the region of the visible light as well as in the region of a UV radiation, and can absorb lights over the entire region of the visible light. For example, the photocatalyst may be active in response to a visible light having a wavelength rage of 380 nm to 780 nm, and specifically exhibit an absorbance of about 20% with respect to a visible light having a wavelength of about 400 nm, such as about 10% with respect to a visible light having a wavelength of about 500 nm.

The photocatalyst is a material that is capable of creating electrons and holes by light energy to generate superoxide anions or hydroxy radicals, such that it can have air cleaning, deodorizing, and antibacterial functions. For example, superoxide anions or hydroxyl radicals generated from the photocatalyst may decompose environmentally harmful substances such as formaldehyde. Further, a high absorption rate of the photocatalyst to visible light can achieve a very high efficiency even under indoor light sources, and therefore no separate UV-supply device may be required.

According to the method for preparing a photocatalyst, the particles of the second metal may be doped evenly inside the pores of the porous film of the first metal oxide.

The present method as described above does not include a heat treatment for reducing the ions of the second metal to form particles. In this reason, the particles can be distributed evenly over a surface of the porous film of the first metal oxide, while forming particulates having a smaller diameter.

The particles of the second metal and the particles of the second metal oxide may have an average diameter of about 1 nm to about 10 nm, specifically, about 1 nm to about 5 nm. The particles of the second metal and the particles of the second metal oxide may be formed in nano-sized particles having a uniform particle size distribution depending on the preparation method for photocatalyst. The photocatalyst uniformly includes the particles of the second metal and the particles of the second metal oxide within the above ranges over the entire surface of the porous film of the first metal oxide, such that the activity efficiency to the visible light can be further improved.

In addition, the photocatalyst may be uniformly dispersed and distributed inside the overall internal pores of the porous film of the first metal oxide. As such, the photocatalyst allows the particles of the second metal and the particles of the second metal oxide to be uniformly dispersed and distributed inside the overall porous film of the first metal oxide, such that the activity efficiency to the visible light can be further improved.

The photocatalyst may have a weight ratio of the sum of the particles of the second metal and the particles of the second metal oxide to the porous film of the first metal oxide of about 0.1:99.9 to about 1:99.

The porous film of the first metal oxide may have a thickness of about 30 nm to about 100 nm.

The second metal may include at least one selected from the group consisting of tungsten, chromium, vanadium, molybdenum, copper, iron, cobalt, manganese, nickel, platinum, gold, cerium, cadmium, zinc, magnesium, calcium, strontium, barium, radium, and combinations thereof.

In order to form the porous film of the first metal oxide, for example, the porous film of the first metal oxide may be formed on a substrate, wherein the substrate may be a glass substrate.

In certain embodiments, the porous film of the first metal oxide may be formed on the substrate by a sol-gel process using a precursor of the first metal oxide. Specifically, the porous film of the first metal oxide may be formed as a crystalline film obtained by coating a solution including a precursor of the first metal oxide as a sol, then drying to form a gel-phase film, followed by heat-treatment.

In certain embodiments, the porous film of the first metal oxide may be coated on a planar substrate as s sol phase obtained by preparing a solution comprising a precursor of the first metal oxide such as metal alkoxide, alcohol, acid, and the like, followed by a hydrolysis, and then a dehydration or a de-alcoholization. The sol-gel process may be carried out in accordance with known process conditions, but is not limited to specific conditions.

After forming the porous film of the first metal oxide, the heat treatment and the crystallization of the first metal oxide allow the first metal oxide to be photoactive.

The heat treatment may be carried out for example at about 500 to about 700°C for about 5 minutes to about 15 minutes. The heat treatment within these ranges allow the first metal oxide of the porous film of the first metal oxide to be crystallized to have a photocatalytic reactivity while preventing an aggregation, thereby controlling its surface area not to get smaller.

The porous film of the first metal oxide formed as above is then dipped into a precursor solution of a second metal, and then the precursor solution of the second metal is uniformly permeated inside the pores of the porous film of the first metal oxide.

The precursor solution of the second metal is a solution comprising an ion of the second metal. The porous film of the first metal oxide is dipped into the precursor solution of the second metal, thereby allowing the ions of the second metal to penetrate into the pores of the porous film of the first metal oxide, and then photo-irradiated, such that the ions of the second metal can be bonded to an inner surface of the pores of the porous film of the first metal oxide. The photo-irradiation may be carried out for example by UV irradiation.

Then, the ions of the second metal bonded as above are reduced again at a later stage, and may be formed on a surface of the inner pores of the porous film of the first metal oxide as particles of the second metal.

In order to reduce the ions of the second metal bonded as above, the porous film of the first metal oxide containing the ions of the second metal inside inner pores is dipped into a solution of alcohol, and then is photo-irradiated. The photo-irradiation may be carried out for example by UV irradiation.

The solution of alcohol may include for example methanol, ethanol, or the like.

When the porous film of the first metal oxide containing the ions of the second metal in its inner pores is dipped into the solution of alcohol, and then is photo-irradiated again, the ions of the second metal are reduced by electrons excited by the photo-irradiation to form the particles. The particles of the second metal formed by the reduction may be formed in a very small size, e.g., several nanometer scales, in their average diameter, and formed uniformly.

According to the preparation method of the photocatalyst, since the particles of the second metal in the precursor solution of the second metal are doped into the first metal oxide formed as a film, they may be easily and uniformly penetrated throughout the interior of the film of the first metal oxide, and evenly dispersed and distributed. The particles of the second metal formed by dipping the ions of the second metal uniformly distributed as above using an alcohol instead of heat treatment may be also uniformly dispersed and distributed throughout the interior of the film of the first metal oxide. Further, as formed herein above, the particles of the second metal are nano-sized particles, and can have a uniform particle size distribution. Forming the particles of the second metal as in the above method allows the photocatalyst to have superior activity efficiency to the visible light as described above.

A precursor compound of the second metal that may be used as the precursor solution of the second metal may be a substance that can be reduced to the second metal by the electrons excited by photo-irradiation, and may include, without limitation, any salt compound soluble in an aqueous solution, such as nitrate, sulfate, chloride, bromide, or the like of the second metal. For example, the precursor compound may include a Cu precursor such as Cu(NO₃)₂, CuSO₄, CuCl₂, CuCl, etc., a Pt precursor such as PtCl₂, PtCl₄, PtBr₂, H₂PtCl₆, K₂(PtCl₄), Pt(NH₃)₄Cl₂, etc., a Au precursor such as AuCl, AuBr, Aul, Au(OH)₂, HAuCl₄, KAuCl₄, KAuBr₄, etc., and a Pd precursor such as (CH₃COO)₂Pd, PdCl₂, PdBr₂, Pdₗ₂, Pd(OH)₂, Pd(NO₃)₂, PdSO₄, etc.

The photo-irradiation may be carried out for example by UV irradiation. Process conditions including photo-irradiation dose, photo-irradiation time period, and the like may be controlled to adjust a doped amount of the second metal in the photocatalyst. For example, the photo-irradiation dose and photo-irradiation time may be increased such that the doped amount of the second metal gets increased.

In another embodiment of the present disclosure, there is provided a photocatalyst prepared by the method for preparing the photocatalyst, including: the porous film of the first metal oxide; and the particles of the second metal formed inside the pores of the porous film of the first metal oxide.

The photocatalyst may be for example applicable to air cleaning, deodorization, and anti-bacterial applications.

Next, the present disclosure will be described in more detail with reference to some examples. It should be understood that these examples are provided for illustration only and art not to be in any way construed as limiting the present disclosure.

### EXAMPLES

### Example 1 Preparation of Pt/TiO₂

10% by weight solution of titanium tetraisopropoxide in isopropyl alcohol was prepared. After stirring 30 minutes, a small amount of concentrated nitric acid was added for hydrolysis. Then, dehydration and de-alcoholization through stirring for 30 minutes resulted in TiO₂ sol.

The resultant was coated on borosilicate glass using a spin coater, and calcined at 600°C for 10 minutes for crystallization of TiO₂ to produce TiO₂ film having a size of 165 mm x 165 mm and a thickness of 50 nm. The TiO₂ film was dipped into 0.01 wt. % aqueous H₂PtCl₆ solution for 30 minutes, and then irradiated using a 20 W UV lamp for 30 minutes to dope Pt into the TiO₂ film. Then, the Pt doped TiO₂ film was dipped into methanol solution for 30 minutes, followed by irradiating using 20 W UV lamp for about 30 minutes to produce a photocatalyst.

### Comparative Example 1

10% by weight solution of titanium tetraisopropoxide in isopropyl alcohol was prepared. After stirring 30 minutes, a small amount of concentrated nitric acid was added for hydrolysis. Then, dehydration and de-alcoholization through stirring for 30 minutes resulted in TiO₂ sol.

The resultant was coated on borosilicate glass using a spin coater, and calcined at 600°C for 10 minutes for crystallization of TiO₂ to produce TiO₂ film having a size of 165 mm x 165 mm and a thickness of 50 nm. The TiO₂ film was dipped into 0.01 wt. % aqueous H₂PtCl₆ solution, and then irradiated using a 20 W UV lamp for about 30 minutes to dope Pt into the TiO₂ film. Then, the Pt doped TiO2 film was heat treated at 600°C for 30 minutes to produce a photocatalyst.

### Experimental Example 1

Pt particle sizes were evaluated from SEM images for the photocatalysts obtained in Example 1 and Comparative Example 1, and the results were summarized in Table 1 below.

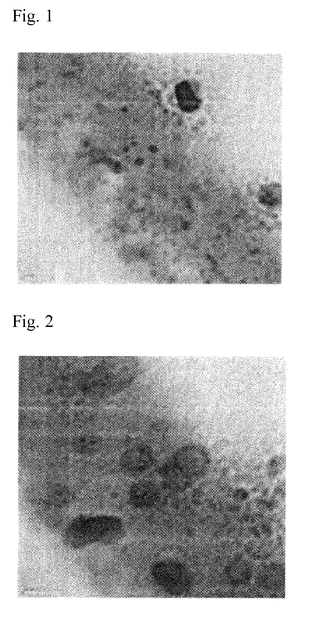

Fig. 1 shows a SEM image for the photocatalyst produced in Example 1, and Fig. 2 shows a SEM image for the photocatalyst produced in Comparative Example 1. It can be found that the Pt particles in Fig. 1 were smaller and more uniformly formed than Fig. 2.

### Experimental Example 2

The removal performance of the photocatalysts in Example 1 and Comparative Example 1 for formaldehyde was evaluated. The photocatalysts produced in Example 1 and Comparative Example 1 were installed in 20 L small chamber (ADTEC Inc.), and then clean air having a formaldehyde concentration of 0.08 ppm was allowed to continuously flow at a flow rate of 167 cc/min to obtain a ventilation number of 0.5 times/hr. A 10 W white fluorescent lamp was used as a light source, and the illuminance was set to 1000 lux. The removal rate of the formaldehyde was calculated by measuring the concentrations before entering the chamber and after passing through the chamber, and then the results were summarized in Table 1 below. The concentrations were analyzed by high performance liquid chromatography (HPLC, Agilent, Inc.) by concentrating the amounts for 10 L using DNPH (2,4-dinitrophenylhydrazine) cartridge.

**Table 1**

| | Pt particle size as formed | Formaldehyde removal rate |
|---|---|---|
| Ex. 1 | 1-2 nm | 75% |
| C. Ex. 1 | 5-7 nm | 50% |

As can be seen from Table 1, we have confirmed that the photocatalyst in Example 1 in which smaller and uniform Pt particles were formed is excellent in the photocatalytic efficiency, thereby providing a high removal rate of formaldehyde.

## Claims

1. A method for preparing a photocatalyst, the method comprising:
forming a porous film of the first metal oxide formed from a first metal oxide by a sol-gel process;
heat-treating the porous film of the first metal oxide to crystallize the first metal oxide;
dipping the porous film of the first metal oxide into a precursor solution of a second metal, followed by a photo-irradiation, such that ions of the second metal is to be penetrated inside pores of the porous film of the first metal oxide; and
dipping the porous film of the first metal oxide containing the ions of the second metal inside the pores into a solution of alcohol, followed by a photo-irradiation, such that the ions of the second metal is to be reduced to form particles of the second metal inside the pores of the porous film of the first metal oxide.

2. The method for preparing a photocatalyst of claim 1, wherein the particles of the second metal formed inside the pores of the porous film of the first metal oxide have an average particle diameter of1 nm to 10 nm.

3. The method for preparing a photocatalyst of claim 1, wherein the first metal oxide contained in the porous film of the first metal oxide comprises at least one selected from titanium oxide, tungsten oxide, zinc oxide, niobium oxide, and combinations thereof.

4. The method for preparing a photocatalyst of claim 1, wherein the second metal comprises at least one metal selected from the group consisting of tungsten, chromium, vanadium, molybdenum, copper, iron, cobalt, manganese, nickel, platinum, gold, cerium, cadmium, zinc, magnesium, calcium, strontium, barium, radium, and combinations thereof.

5. The method for preparing a photocatalyst of claim 1, wherein the photocatalyst has a weight ratio of the particles of the second metal to the porous film of the first metal oxide of 0.1:99.9 to 1:99.

6. The method for preparing a photocatalyst of claim 1, wherein the photocatalyst is photoactive under a visible light having a wavelength range of 380 nm to 780 nm.

7. The method for preparing a photocatalyst of claim 1, wherein the porous film of the first metal oxide is formed in a thickness of 30 nm to 100 nm.

8. The method for preparing a photocatalyst of claim 1, wherein the photo-irradiation is carried out by UV irradiation.

9. A photocatalyst prepared by the method for preparing a photocatalyst according to any one of claims 1 to 8, comprising:
the porous film of the first metal oxide; and
the particles of the second metal formed inside the pores of the porous film of the first metal oxide.

10. The photocatalyst of claim 9, wherein the particles of the second metal formed inside the pores of the porous film of the first metal oxide have an average particle diameter of 1 nm to 10 nm.

11. The photocatalyst of claim 9, wherein the first metal oxide contained in the porous film of the first metal oxide comprises at least one selected from titanium oxide, tungsten oxide, zinc oxide, niobium oxide, and combinations thereof.

12. The photocatalyst of claim 9, wherein the second metal comprises at least one metal selected from the group consisting of tungsten, chromium, vanadium, molybdenum, copper, iron, cobalt, manganese, nickel, platinum, gold, cerium, cadmium, zinc, magnesium, calcium, strontium, barium, radium, and combinations thereof.

13. The photocatalyst of claim 9, wherein the photocatalyst has a weight ratio of the particles of the second metal to the porous film of the first metal oxide of 0.1:99.9 to 1:99.

14. The photocatalyst of claim 9, wherein the photocatalyst is photoactive under a visible light having a wavelength range of 380 nm to 780 nm.

15. The photocatalyst of claim 9, wherein the porous film of the first metal oxide has a thickness of 30 nm to 100 nm.

16. The photocatalyst of claim 9, which is applicable to air cleaning, deodorizing, or antibacterial applications.
